# EUROPEAN PATENT APPLICATION

(11) **EP 1 733 751 A1**
(43) Date of publication of application: **20.12.2006**
(21) Application number: 06270056.2
(22) Date of filing: 12.06.2006
(51) Int. Cl.: A61M 16/16

(54) **Humidification chamber for use in a breathing circuit**

(30) Priority: 15.06.2005 GB 0512083
(71) Applicant: Intersurgical AG, Vaduz (LI)
(72) Inventor: Fernando, Keethan, Berkshire RG41 1EP (GB); Payne, Simon Robert, Godalming Surrey GU8 4LU (GB)
(74) Representative: Jones, Stephen Anthony

(57) **Abstract**

A humidification chamber (10) for use in the humidification of gases intended for inhalation is disclosed. The humidification chamber (10) is adapted to contain a volume of liquid (20), and comprises a gas inlet port (30) and a gas outlet port (30) such that gases flow, in use, through the humidification chamber (10). At least one baffle (50) extends downwardly from an interior surface of the humidification chamber (10). The at least one baffle (50) has a lower edge that is situated, in use, above an exposed surface of the liquid (20), and acts, in use, to deflect gases flowing from the gas inlet port (30) to the gas outlet port (30) downwardly towards the exposed surface of the liquid (20).

## Description

This invention relates to humidification chambers, and in particular to humidification chambers for use in a breathing circuit to humidify gases before inhalation.

The inhalation of gases lacking sufficient moisture may damage or irritate the respiratory tract, and/or desiccate essential secretions, of patients whose supraglottic airways have been bypassed. Gases within a breathing circuit are therefore usually humidified before inhalation using a suitable humidification chamber.

Conventional humidification chambers generally contain a volume of water, and have two ports through which gases enter and exit the humidification chamber, and means for heating the water. Furthermore, many humidification chambers include means for replacing water that is lost from the humidification chamber so as to maintain the level of the water relatively constant. Such means typically takes the form of a liquid inlet valve having a float actuator, in which the rise and fall of the float actuator, in use, acts to open and close the valve so as to maintain the level of the water in the humidification chamber relatively constant.

Conventional humidification chambers having liquid inlet valves may suffer from a number of disadvantages. For instance, the float actuator may significantly reduce the surface area of the water that is exposed to the gas flow, and also the flow path between the inlet and outlet may be situated substantially within an upper part of the humidification chamber and hence be distanced from the surface of the water. Both these problems may significantly reduce the humidification efficiency of conventional humidification chambers.

There has now been devised an improved humidification chamber and an improved breathing circuit which overcome or substantially mitigate the above-mentioned and/or other disadvantages associated with the prior art.

According to a first aspect of the invention, there is provided a humidification chamber for use in the humidification of gases intended for inhalation, the humidification chamber being adapted to contain a volume of liquid, and comprising a gas inlet port and a gas outlet port such that gases flow, in use, through the humidification chamber, wherein at least one baffle extends downwardly from an interior surface of the humidification chamber, said at least one baffle having a lower edge that is situated, in use, above an exposed surface of the liquid, and said at least one baffle acting, in use, to deflect gases flowing from the gas inlet port to the gas outlet port downwardly towards the exposed surface of the liquid.

The humidification chamber according to the invention is advantageous principally because the baffle(s) guide gases flowing through the humidification chamber towards an exposed surface of the liquid contained in the chamber, thereby improving the humidification efficiency of the humidification chamber. Furthermore, the baffie(s) create turbulent flow, and also increase the period of time for which the gases remain within the humidification chamber, thereby further improving the humidification efficiency of the humidification chamber.

The humidification chamber may have any number of baffles, but the at least one baffle preferably forms at least one continuous barrier that extends across an upper portion of the humidification chamber.

The or each baffle preferably comprises a web of material that is integrally formed with an upper wall of the humidification chamber. The web of material is preferably of narrow thickness, most preferably less than 3mm thickness, in order to facilitate the formation of turbulent flow about the lower edge of the baffle. The lower edge of the or each baffle is preferably orientated generally horizontally, in use.

The or each baffle may extend between first and second points on the interior surface of a side wall of the humidification chamber, so as to form a continuous barrier that extends across an upper portion of the humidification chamber. Alternatively, however, the humidification chamber may have one or more continuous barriers that extend across an upper portion of the humidification chamber, each barrier comprising a pair of baffles with each baffle extending between an interior surface of the side wall of the humidification chamber and an external surface of a liquid inlet or liquid inlet valve. In a presently preferred embodiment, the humidification chamber comprises a first continuous barrier formed adjacent to the entrance into the gas inlet port and a second continuous barrier formed adjacent to the entrance into the gas outlet port.

The humidification chamber is preferably adapted such that gases flow, in use, through the space defined between the exposed surface of the liquid, the surfaces of the baffie(s), and the interior surface of the walls of the humidification chamber.

The gas inlet port and gas outlet port are preferably formed in an upper wall of the humidification chamber, and preferably comprise upstanding tubular connectors that are adapted for connection to conventional respiratory connectors and tubing. The gas inlet and outlet ports are preferably arranged along a diameter of the upper wall of the humidification chamber, and are preferably situated a similar distance from the centre of the upper wall.

The gas inlet and outlet ports preferably each include a baffle that prevents gas flowing unhindered into the port. In particular, each port preferably includes a baffle that either deflects incoming gas towards a side wall of the chamber, or causes outgoing gas to flow into the port from part of the chamber that is adjacent to its side wall. For instance, each port may include an extension that extends into the chamber, the extension having an end wall and an opening in a side wall, and the opening facing a side wall of the humidification chamber, such that gas flowing, in use, into the chamber through the port is deflected away from the centre of the chamber and towards the interior surface of the side wall of the chamber.

The liquid is normally water, or a suitable aqueous solution. During use, the volume of liquid within the humidification chamber will gradually reduce as the gases flowing through the chamber are humidified. The humidification chamber preferably therefore includes a liquid inlet for introducing replacement liquid into the humidification chamber during use. Most preferably, the volume of fluid within the humidification chamber is maintained above a minimum acceptable level.

The liquid inlet is preferably formed in an upper wall of the humidification chamber, most preferably on a straight line extending between the gas inlet port and the gas outlet port, and is preferably adapted for engagement with a suitable supply conduit.

The humidification chamber may be adapted so that a user manually introduces liquid into the humidification chamber during use. However, the humidification chamber may include a liquid inlet valve for controlling the introduction of liquid into the humidification chamber during use. The liquid inlet valve may include a float actuator that is movable, in use, in response to changes in the level of the liquid within the humidification chamber, so that the liquid inlet valve has an open configuration in which liquid is able to flow through the liquid inlet valve into the humidification chamber when the volume of liquid is below a predetermined level, and a closed configuration in which liquid is unable to flow through the liquid inlet valve into the humidification chamber when the volume of liquid is at or above the predetermined level.

Where the humidification chamber includes a liquid inlet valve with a float actuator, the liquid inlet valve preferably comprises a liquid inlet that extends into the humidification chamber, and the float actuator is preferably adapted to seal the liquid inlet in the closed configuration. The float actuator preferably comprises a valve cushion on an upper surface of float actuator that is adapted to seal the liquid inlet in the closed configuration. A guiding sleeve may be provided for guiding the float along a linear path into, and out of, engagement with the liquid inlet. Alternatively, the float may be pivotally mounted relative to the liquid inlet.

The humidification chamber preferably comprises an upper portion that is formed of plastic material, most preferably by injection moulding, and a base formed of a good heat conductor, such as a suitable metal, that together define an enclosure for containing the liquid. The base is preferably generally circular in shape, and the upper portion preferably has a generally cylindrical side wall.

According to a further aspect of the invention, there is provided a breathing circuit comprising a humidification chamber as described above.

The invention will now be described in greater detail, by way of illustration only, with reference to the accompanying drawings, in which
Figure 1 is a perspective view of a first embodiment of a humidification chamber according to the invention, showing some hidden detail;
Figure 2 is a plan view of the humidification chamber of Figure 1, showing some hidden detail;
Figure 3 is a cross-sectional view along the line III-III in Figure 2; and
Figure 4 is a cross-sectional view along the line IV-IV in Figure 2.

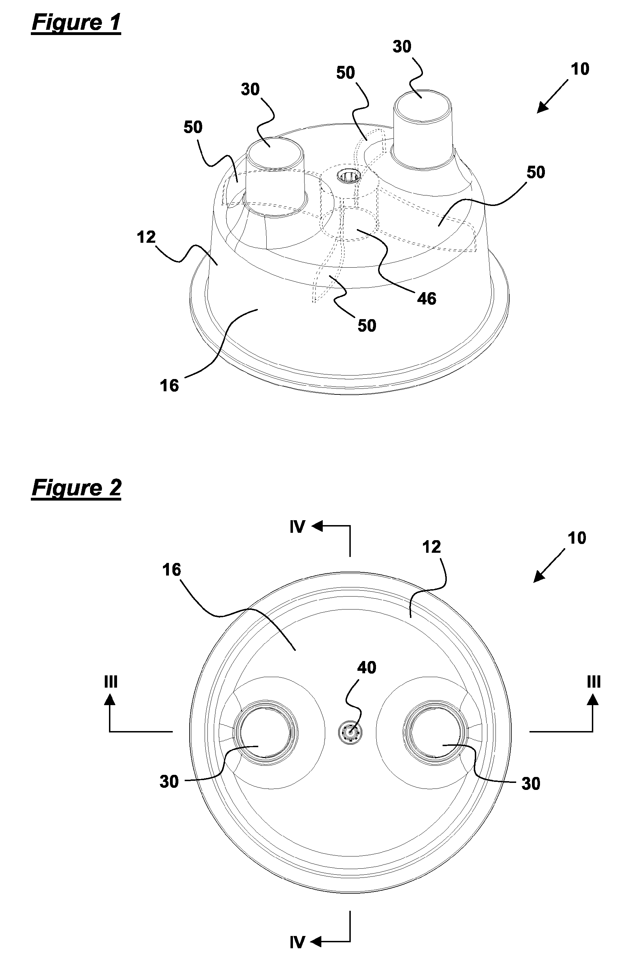

Figures 1 to 4 show a first embodiment of a humidification chamber according to the invention, which is generally designated 10. The humidification chamber 10 comprises an upper portion 12 that is injection moulded in a transparent plastics material, and a metal base 14 fixed to an open lower end of the upper portion 12. The upper portion 12 and the base 14 of the humidification chamber 10 cooperate to define an enclosure 16 for containing, in use, a volume of water 20.

The upper portion 12 of the humidification chamber 10 comprises a generally cylindrical, but slightly tapered, side wall that is fixed at its lower end to the periphery of the base 14, and an upper wall that has the general shape of a shallow dome. Two inlet/outlet ports 30 that have the form of 22mm tubular connectors extend upwardly from the upper wall of the humidification chamber 10. The ports 30 are arranged along a diameter of the upper wall, and are spaced equal distances from the centre of the upper wall.

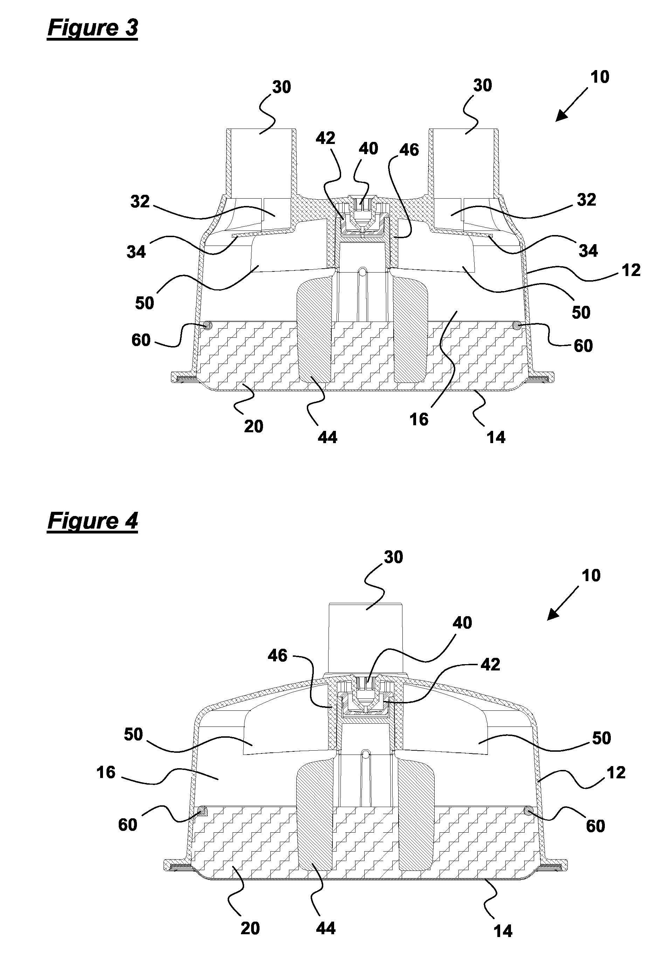

Each port 30 includes a hemi-cylindrical extension 32 (see Figure 3) that extends into the enclosure 16 of the humidification chamber 10, and a circular baffle 34 that extends generally horizontally from the lower end of the extension 32 and faces the open lower end of the corresponding port 30. The extension 32 and baffle 34 of each port 30 are arranged so that gas flowing, in use, into the enclosure 16 through the port 30 is deflected away from the centre of the enclosure 16 and towards the interior surface of the side wall of the humidification chamber 10. Similarly, gas flowing into the port 30 from the enclosure 16 flows into the port 30 from a part of the enclosure 16 that is adjacent to the side wall of the humidification chamber 10.

A liquid inlet valve, which includes a valve cushion 42 and a float actuator 44, is formed in the centre of the humidification chamber 10. In particular, the upper wall of the humidification chamber 10 is formed with a centrally positioned and downwardly extending liquid inlet 40 having an upper opening in the upper wall of the humidification chamber 10, and a smaller, lower opening at the lower end of the liquid inlet 40. The interior surface of the liquid inlet 40 includes formations that enable cooperation with a suitable liquid conduit, such that the liquid inlet 40 communicates with a source of water during use.

The upper wall of the humidification chamber 10 further includes a centrally positioned and downwardly extending guiding sleeve 46 of cylindrical shape, which is of greater diameter than the liquid inlet 40. Eight longitudinal ribs are provided on the interior surface of the guiding sleeve 46 at equiangularly spaced positions, so as to form channels for the water 20 being supplied through the liquid inlet 40.

The float 30 comprises an upper portion and a lower portion, which are integrally formed in a plastics material using an injection moulding process that is described in published European patent application EP 1366881. The upper portion of the float 44 is a generally cylindrical tube with relatively thin walls and a diameter that increases gradually and slightly from an open upper end to a open lower end. A circular partition extends across the interior of the upper portion so as to define a cylindrical recess in the upper surface of the float 44. The lower end of the upper portion has an outwardly-extending, curved lip which extends into the upper end of the lower portion. In addition, the upper portion includes four openings, at equiangularly spaced positions, in a lower part of its wall below the circular partition.

The lower portion of the float 44 is also generally cylindrical in form, but has walls of greater thickness than those of the upper portion of the float 44. The plastics material of the lower portion has a foam-like structure with many pockets of gas trapped within the plastics material. The float 44 is located within the enclosure 16 such that the lower end of the float 44 rests on the base 14 of the enclosure 16 until a volume of water 20 is formed, and the majority of the upper portion of the float 44 is received within the guiding sleeve 46.

A valve cushion 42, which is typically formed from elastomeric material, has the form of a cup and is received with an interference fit within the cylindrical recess in the upper surface of the float 44. The open upper end of the valve cushion 42 has an outwardly extending flange that rests upon the rim of the upper portion of the float 44, and the base of the valve cushion 42 lies immediately below the lower opening of the liquid inlet 40.

As shown most clearly in Figure 1, the humidification chamber 10 includes four baffles 50 that are each formed integrally with the upper portion 12 of the humidification chamber 10, and each extend downwardly from the interior surface of the upper wall. Each baffle 50 is arcuate in its horizontal dimension, and extends horizontally between an exterior surface of the guiding sleeve 46 and an interior surface of the side wall of the humidification chamber 10. The baffles 50 are arranged so as to cooperate with the guiding sleeve 46 to form an arcuate barrier about each of the inlet/outlet ports 30. The two barriers formed by the baffles 50 and the guiding sleeve 46 each prevent the unhindered flow of gas through an upper part of the enclosure 16. Each barrier has a substantially horizontal lower edge that is disposed above an exposed surface of the water 20 during use. In this way, each barrier deflects gases flowing through an upper portion of the enclosure 16 downwardly towards the exposed surface of the water 20 during use, and also acts to create turbulent flow, thereby increasing the humidifying efficiency of the humidification chamber 10.

Finally, the humidification chamber 10 includes a liquid level indicator that enables a user to readily ascertain the level of the water 20. This liquid level indicator comprises an annular float 60, and is described in published European patent application EP 1347797.

Before use, a liquid conduit (not shown in the Figures) is connected at one end to a source of water, and at the other end to the liquid inlet 40. Water 20 flows through the liquid inlet 40, fills the valve cushion 42, and then flows through the channels defined by the longitudinal ribs of the guiding sleeve 46 and down the exterior surface of the float 44 onto the base 14 of the enclosure 16. The enclosure 16 therefore begins to fill with water 20. When the water 20 within the enclosure 16 reaches a certain level, the float 44 is lifted relative to the enclosure 16 by its buoyancy. The float 44 is held in an upright position by the guiding sleeve 46. The float 44 will continue to rise until the water 20 reaches a sufficient level for the valve cushion 42 to be forced against the lower opening of the liquid inlet 40 with enough force to prevent the inflow of water 20 through the lower opening.

If the level of the water 20 falls, the float 44 will fall relative to the enclosure 16 until the valve cushion 42 is removed from the lower opening of the liquid inlet 40 and water 20 is allowed to flow into the enclosure 16 through the liquid inlet 40. In this way, the level of the water 20 is maintained relatively constant during use. In addition, the openings in the float 44 prevent the buoyancy of the float 44 being affected by air becoming trapped between the surface of the water 20 and the interior surface of the lower portion of the float 44.

The humidification chamber 10 is connected to a breathing circuit by attaching a gas inlet conduit (not shown in the Figures) to one of the inlet/outlet ports 30, and attaching a gas outlet conduit to the other inlet/outlet port 30. Finally, a heat source (not shown in the Figures) is placed in contact with the base 14 of the humidification chamber 10, so as to heat the water 20 to a desired temperature.

In use, gases intended for inhalation by a patient are supplied to the gas inlet conduit under positive pressure. The pressure differential created between the gas inlet conduit and the gas outlet conduit causes gases to flow from the gas inlet conduit, through the enclosure 16 of the humidification chamber 10, to the gas outlet conduit. This causes water vapour within the chamber 30 to be entrained in the flow of gas through the humidification chamber 10, so that the gas within the gas outlet conduit has an increased humidity relative to the gas with the gas inlet conduit.

In particular, gases flow along the gas inlet conduit, and through the corresponding port 30 of the humidification chamber 10. The gases are deflected by the extension 32 and baffle 34 of the port 30 towards the side wall of the humidification chamber 10. The gases will then flow, guided by the side wall of the humidification chamber 10, in the general direction of the port 30 to which the gas outlet conduit is attached. However, the barriers formed by the baffles 50 and the guiding sleeve 46 of the humidification chamber 10 deflect the gases downwardly towards an exposed surface of the water 20, and also act to create a turbulent flow about their lower edges. This directs the gases into closer proximity with exposed surfaces of the water 20 and creates turbulent flow, thereby improving the humidification efficiency of the humidification chamber 10.

Finally, the gases flow around the extension 32 and baffle 34 of the port 30 to which the gas outlet conduit is attached, through the port 30 and the gas outlet conduit, before being supplied to the patient for inhalation.

A second embodiment of a humidification chamber according to the invention is identical to the first embodiment 10, save for the omission of the liquid inlet valve, including the valve cushion 42 and the float actuator 44. Hence, the second embodiment of the humidification chamber according to the invention includes the same upper portion 12, metal base 14, enclosure 16, two inlet/outlet ports 30, port extension 32 and baffle 34, liquid inlet 40, guiding sleeve 46, baffles 50, and fluid level indicator 60, of the first embodiment 10 that are shown in Figures 1 to 4. The second embodiment functions in an identical manner to the first embodiment 10, save for the need to manually introduce replacement water 20 into the humidification chamber through the liquid inlet 40 during use.

## Claims

1. A humidification chamber (10) for use in the humidification of gases intended for inhalation, the humidification chamber (10) being adapted to contain a volume of liquid (20), and comprising a gas inlet port (30) and a gas outlet port (30) such that gases flow, in use, through the humidification chamber (10), wherein at least one baffle (50) extends downwardly from an interior surface of the humidification chamber (10), said at least one baffle (50) having a lower edge that is situated, in use, above an exposed surface of the liquid (20), and said at least one baffle (50) acting, in use, to deflect gases flowing from the gas inlet port (30) to the gas outlet port (30) downwardly towards the exposed surface of the liquid (20).

2. A humidification chamber (10) as claimed in Claim 1, wherein the at least one baffle (50) forms at least one continuous barrier that extends across an upper portion of the humidification chamber (10).

3. A humidification chamber (10) as claimed in Claim 1 or Claim 2, wherein the or each baffle (50) comprises a web of material that is integrally formed with an upper wall of the humidification chamber (10).

4. A humidification chamber (10) as claimed in Claim 3, wherein the web of material is of less than 3mm thickness.

5. A humidification chamber (10) as claimed in any preceding claim, wherein the lower edge of the or each baffle (50) is orientated generally horizontally, in use.

6. A humidification chamber (10) as claimed in any preceding claim, wherein the or each baffle (50) extends between first and second points on the interior surface of a side wall of the humidification chamber (10), so as to form a continuous barrier that extends across an upper portion of the humidification chamber.

7. A humidification chamber (10) as claimed in any one of Claims 1 to 5, wherein the humidification chamber (10) has one or more continuous barriers that extend across an upper portion of the humidification chamber (10), each barrier comprising a pair of baffles (50) with each baffle (50) extending between an interior surface of the side wall of the humidification chamber (10) and an external surface of a liquid inlet (40) or liquid inlet valve (42,44,46).

8. A humidification chamber (10) as claimed in Claim 6 or Claim 7, wherein the humidification chamber (10) comprises a first continuous barrier formed adjacent to the entrance into the gas inlet port (30) and a second continuous barrier formed adjacent to the entrance into the gas outlet port (30).

9. A humidification chamber (10) as claimed in any preceding claim, wherein the gas inlet and outlet ports (30) each include a baffle (50) that prevents gas flowing unhindered into the port (30).

10. A humidification chamber (10) as claimed in Claim 9, wherein each port (30) includes a baffle (34) that either deflects incoming gas towards a side wall of the chamber (10), or causes outgoing gas to flow into the port (30) from part of the chamber (10) that is adjacent to its side wall.

11. A humidification chamber (10) as claimed in any preceding claim, wherein each port (30) includes an extension (32) that extends into the chamber (10), the extension (32) having an end wall (34) and an opening in a side wall, and the opening facing a side wall of the humidification chamber (10), such that gas flowing, in use, into the chamber (10) through the port (30) is deflected away from the centre of the chamber (10) and towards the interior surface of the side wall of the chamber (10).

12. A breathing circuit comprising a humidification chamber (10) as claimed in any preceding claim.
